# EUROPEAN PATENT SPECIFICATION

(11) **EP 0 630 219 B1**
(45) Date of publication and mention of the grant of the patent: **04.06.1997**
(21) Application number: 93906460.6
(22) Date of filing: 03.03.1993
(51) Int. Cl.: A61F 5/447, A61F 5/44

(54) **COLLECTION BAGS AND METHOD FOR PRODUCING SUCH BAGS**
SAMMELBEUTEL SOWIE VERFAHREN ZUM HERSTELLEN SOLCHER BEUTEL
SACS COLLECTEURS ET LEURS PROCEDES DE FABRICATION

(30) Priority: 04.03.1992 DK 289/92
(43) Date of publication of application: 28.12.1994
(73) Proprietor: COLOPLAST A/S, DK-3060 Espergaerde (DK)
(72) Inventor: ENEVOLDSEN, John, DK-2610 Rodovre (DK); TANGHOJ, Allan, DK-2000 Frederiksberg (DK)
(74) Representative: Christiansen, Ejvind
(86) International application number: DK9300077
(87) International publication number: WO9317643

(56) References cited:
- DK-B- 113 595
- DK-B- 135 928
- DK-B- 149 402
- US-A- 2 741 247

## Description

The present invention concerns collection bags for use by urine-incontinent or ostomy-operated individuals, said collection bags having at least one inlet opening and optionally one or more discharge openings.

Collection bags of the above-mentioned type may be formed from a sheet tube or a sheet member which is folded centrally in longitudinal or transverse directions to form two outer sheet walls, the open ends or edges of the sheet tube or the sheet member being closed by joining, preferably by glueing or welding.

The collection bag which has been used most extensively for many years is a bag consisting of two weldable sheets which, apart from openings for inlet and discharge, are welded together along their peripheries to form a cavity for the collection of urine and/or faeces.

Collection bags of this simple design operate excellently in reality. However, such a bag is not particularly discrete, because when used it bulges very much and is very difficult to conceal for the user particularly in a filled or almost filled state. Furthermore, the collected human vastes, if just a little liquid, will have a great tendency to lap when the user moves about, thereby giving unpleasant sounds that may have a considerable inhibitory effect on the user.

In spite of these problems collection bags of the above-mentioned design are still used.

It has previously been attempted to solve the above-mentioned problems by manufacturing collection bags of a flatter shape. Generally, two types of collection bags are known today which essentially have properties reducing the tendency of the bag contents to lap, and which do not bulge so much as the first-mentioned simple bag.

The first type of these "flatter" collection bags has two sheet walls optionally consisting of two weldable sheets which are welded together along the periphery in the same manner as collection bags of the simple design. The two sheet walls are additionally welded together at one or more spots or in one or more lines, e.g. a longitudinal central weld. This type of collection bag has a relatively flat shape which makes it easier for the user to conceal it under the clothes, and the flat shape of the bag as well as the division into chambers entail that the tendency of the bag contents to lap is reduced considerably. A collection bag of this type is known e.g. from DK published application 113 595.

Thus, such a collection bag of the last-mentioned type does not bulge in one big bulge when the bag is filled or almost filled, but bulges instead in several small, but nevertheless considerable bulges, which imparts a very curved surface to the bag. This entails that the space occupied by the bag under the user's clothes is utilized very poorly, because the effective volume of the bag, i.e. the volume which the bag can contain, is considerably smaller than the volume or the space occupied by the bag under the user's clothes. Such bags therefore rarely have an effective volume which is particularly great, since, otherwise, the bag would take up unacceptably much space under the user's clothes. Additionally, when the bag is filled, the spot or line welds are loaded by oppositely directed forces which will tend to tear the welds apart. If the bag is additionally subjected to an impact or a blow, this causes a pull in the spot or line welds, which additionally increases the risk of the weld being torn apart. It is a very unpleasant feeling for the user when a weld suddenly fails, and at worst tearing of a weld may cause the bag to leak.

The second type of the "flatter" collection bags consists of four or more sheet layers, all of which have the same peripheral circumference, and which are welded together along their peripheries so that all the sheet layers, both the outer and the inner ones, have the same extent in area. The two central sheets moreover have a plurality of longitudinal welds arranged at intervals side by side. In the space each of the two intermediate sheets is welded together with the contacted outer sheet in longitudinal lines. The collection bag is thus divided into a plurality of longitudinal bag sections which are interconnected via openings in the intermediate sheets. Collection bags of this type are described in e.g. DK published application 135 928 as well as in the applicants' own DK patent specification 149 402.

The last-mentioned collection bag substantially has the same properties as the collection bags with walls welded together in one or more spots or lines, but the bag surface is somewhat less curved. The intermediate sheets thus form some intermediate walls which, when the bag is full, cause the outer sheet walls in the lines where the intermediate sheets are welded to these to be kept apart at a distance which is considerably smaller than the distance of the outer sheet walls between the weld lines, the inner sheets causing a transverse pull in the bag owing to their zigzag configuration, which necessarily causes the outer sheet walls to bulge between the longitudinal welds. The effective volume of this collection bag is therefore likewise unacceptably small with respect to the space occupied by the bag under the user's clothes, and the bag is moreover very expensive to manufacture, because not only is considerably more sheet material to be used, but also a complicated welding tool as well as a manufacturing process which is time-consuming and difficult in terms of handling.

The object of the present invention is to provide collection bags which have an essentially flat or plane shape, and which satisfactorily prevent liquid contents from lapping, and which have "safe" welds, and which also have satisfactorily effective volumes with respect to the shape occupied by the bags under the user's clothes.

This object is achieved by the collection bags of the invention which are of the type defined in the introductory portion of claim 1, and which are characterized by the features defined in the characterizing portion of claim 1.

Thus, a collection bag according to the invention comprises one or more inner sheet members which are folded in a suitable configuration, e.g. in a longitudinal fold, a longitudinal double fold or a fold about themselves to form a tube.

Thus, a collection bag according to the invention has one or more intermediate walls which are formed by the inner sheet member or members, and which determine the maximum distance between the two outer sheet walls in the spots or lines where the inner sheet member or members are attached to these, said inner sheet member or members causing no transverse tension in the bag. A collection bag according to the invention thus has a relatively flat shape and a surface which is substantially plane. Thus, the invention provides a collection bag which has an optimum volume with respect to the space occupied by the bag under the user's clothes.

The fold or folds of an inner sheet member between the joints of the sheet member to the one and the other, respectively, of the two sheet walls are to be free. A free fold means that no other tension may occur in the fold than the tension caused by the sheet walls at the attachment points or lines to the inner sheet member. Two sheet members welded together along a side edge to form a free intermediate wall is thus considered as being one sheet member.

The inner sheet member or members are preferably joined with the inner sides of the outer sheet walls by welding, but they may also be attached in another manner, e.g. by glueing, in particular hot melt glueing.

The inner sheet member or members may additionally be attached to each other or to itself.

The joints may be provided in spots or lines, preferably longitudinal lines.

The inner sheet members may have perforations or holes through which the bag contents can pass. Such holes or perforations are advantageous in particular if the inner sheet members are attached to the outer walls at or in the lower edge of the bag.

The outer sheet walls may have one or more inward folds in or at their side edges, as known from US patent specification 2 741 247. These inward folds may e.g. be provided as one or more pleats in one sheet wall.

A collection bag according to the invention having such pleats or inward folds in or at the side edges thus have "high" edges, which entails that when filled the bag has substantially the same thickness along its entire surface extent.

The collection bags according to the invention can moreover be manufactured in a relatively simple and inexpensive manner, and only such an amount of sheets as is functionally necessary is used.

The invention moreover concerns methods for producing the collection bags of the invention, which methods are relatively uncomplicated and rapid and moreover do not present any serious difficulties in terms of handling.

The methods of the invention, which are of the type defined in the introductory portion of claim 11, are characterized by the features defined in the characterizing portion of claim 11.

A first preferred embodiment of a collection bag according to the invention comprises an inner tubular sheet member folded longitudinally. The inner sheet member is welded to the one and the other, respectively, outer sheet in longitudinal rows of spots or lines. The inner sheet member may be tubular already before being welded to the outer sheets, or the sheet member may be folded so as to form the tube, while being welded to the outer sheets.

A second embodiment of a collection bag according to the invention comprises an inner sheet member which, alternately with lateral gaps, is welded to the inner sides of the one and other, respectively, of the outer sheet walls in longitudinal rows of spots or lines, the inner sheet member having a free double fold between each weld line when the bag is empty and thus flat.

These and other preferred embodiments are described more fully below with reference to the drawing, in which
fig. 1 is a perspective view of a first preferred embodiment of a collection bag according to the invention, where the upper portion of the bag has been cut away, and where the bag has the shape it assumes when filled,
fig. 2 is a cross-section of the embodiment shown in fig. 1,
fig. 3 is a cross-section of a variant of the first preferred embodiment of a collection bag according to the invention,
fig. 4 shows the collection bag of fig. 3 after welding, where the bag has the shape it assumes when filled,
fig. 5 is a cross-section of a second variant of the first preferred embodiment of a collection bag according to the invention, seen at the welding situation,
fig. 6 is a cross-section of a third variant of the first preferred embodiment of a collection bag according to the invention, seen immediately before the welding situation,
fig. 7 is a cross-section of a second embodiment of a collection bag according to the invention in a half-filled, horizontal state, and
fig. 8 is a cross-section of a third preferred embodiment of a collection bag according to the invention in a half-filled, horizontal state.

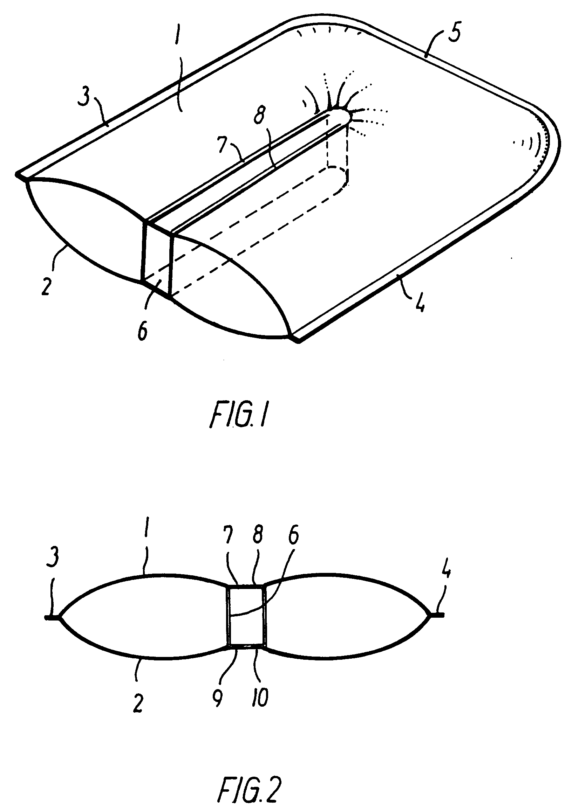

The collection bag shown in figs. 1 and 2 consists of two outer sheet walls (1, 2), which have two welded side edges (3, 4) a lower welded edge (5) and an upper welded edge (not shown). In the upper edge or in one of the two outer sheet walls the upper portion (not shown) of the bag has an inlet opening in which a tube member or the like is attached if it is a urine-incontinens bag, and if it is an ostomy bag, an adhesive layer, a coupling ring as described e.g. in the applicants' own patent publications WO 91/01118 and WO 91/01119, or the like is arranged on the outer side of the sheet wall along the periphery of the opening. A reflux valve is optionally arranged after the inlet opening in a generally known manner. Likewise, the lower portion of the bag may include one or more discharge openings that can be opened and closed, e.g. by means of a valve system. Particularly useful is the valve system mentioned in the applicants' own Danish patent application having the same filing date as the present application. The above-mentioned discharge openings have been omitted for clarity. The collection bag has an inner sheet member which has the shape of a tube (6). The tube is welded to the inner side of the outer sheet wall (1) in two longitudinal weld lines (7, 8) and to the inner side of the outer sheet wall (2) likewise in two longitudinal weld lines (9, 10).

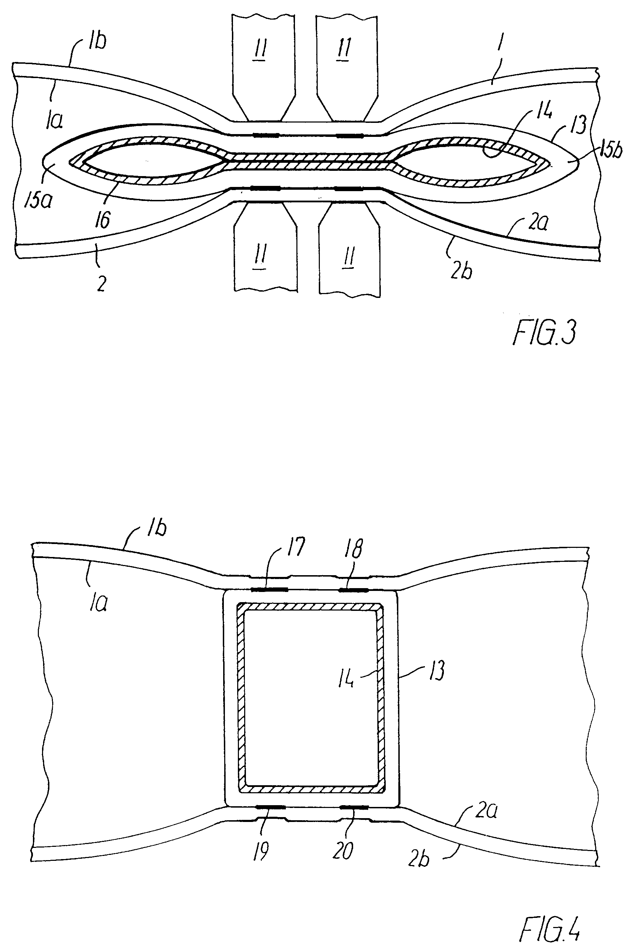

Figs. 3 and 4 show in outline how a first variant of the collection bag shown in figs. 1 and 2 is produced, and thus illustrate the appearance of the characteristic inner sheet members when the bag is empty and filled, respectively. The inner sheet tube (16) consists of a sheet laminate of two different plastics materials (13, 14). The inner layer (14) is not weldable to itself at the given welding parameters, the outer layer (13) being welded to the inner sides (1a, 2a) of the two outer sheet walls (1, 2). If the outer sheet walls (1, 2) of the bag consist of polyethylene, the sheet laminate may e.g. be a laminate of polyethylene and polyester, the polyester constituting the non-weldable layer (14), and the sheet tube (16) being welded to the inner sides (1a, 2a) of the outer sheet walls by hot welding. Another example is a bag having outer sheet walls (1, 2) of PVC, the sheet laminate being a laminate of PVC and polyethylene, the non-weldable layer (14) being constituted by the polyethylene layer, and the tube (16) being attached to the inner sides (1a, 2a) of the outer sheet walls by high frequency welding. It appears from fig. 3 how the welding tool (11) presses against the outer sides (1b, 2b) of the outer sheet walls, thereby causing the outer layer (13) of the tube to be welded to the inner sides (1a, 1b) of the outer sheet walls in the weld lines (17, 18, 19 and 20). The welding tool may optionally be designed such that the two outer sheet walls are simultaneously welded together along their peripheries. In the welding situation shown in fig. 3 the collection bag is empty, and the tube (16) has two free longitudinal folds (15a, 15b) between the weld lines (17, 18) of the tube to the inner side (1a) of one outer sheet wall and the opposed weld lines (19, 20) to the inner side (2a) of the other outer sheet wall. Fig. 4 shows the section of the collection bag after welding in a filled state, and it will be seen that the folds (15a, 15b) shown in fig. 3 are extended between their weld lines (17, 19) and (18, 20), respectively.

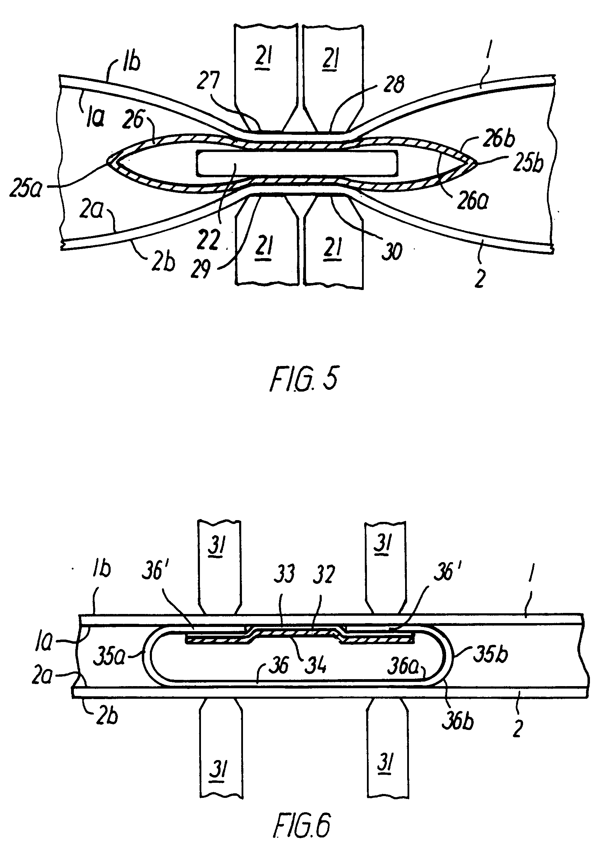

Fig. 5 shows in outline how a second variant of the collection bag shown in figs. 1 and 2 is produced. The inner sheet tube (26) has inner and outer surfaces (26a, 26b). The outer surface (26b) is welded to the inner surfaces (1a, 2a) of the outer sheet walls in longitudinal weld lines (27, 28, 29, 30) by means of the welding tool (21), which presses against the outer surfaces (1b, 2b) of the outer sheet walls, a welding preventing element (22) being arranged loosely, optionally temporarily, inside the tube (26), said element (22) preventing the inner side (26a) of the sheet tube from being welded to itself in the process. Like in fig. 3, it will be seen that the sheet tube (26) has two free longitudinal folds (25a, 25b) which will be stretched when the finished bag is filled. The bag out-lined in fig. 5 is frequently produced in two steps, the outer sheet walls (1, 2) being welded together along their peripheries in a second welding step after the loosely arranged, welding preventing element (22), which in that case is just arranged temporarily, has been removed. Where the loosely arranged, welding preventing element (22) is just mounted temporarily, the welding preventing element (22) may suitably be part of the welding tool.

Fig. 6 shows in outline how a third variant of the collection bag shown in figs. 1 and 2 is produced. The bag section is seen immediately before the welding process. The two outer sheet walls (1, 2) have interposed between them an inner sheet member (36) which is folded about itself in two longitudinal folds (35a, 35b). The sheet member (36) is retained in this position by means of a self-adhesive tape (32) which is partly in adhesive contact with the inner side (36a) of the sheet member along the longitudinal edges (36') of the sheet member and partly in adhesive contact with the inner side (1a) of one outer sheet wall, so that the sheet member (36) and the tape (32) together form a tube. The tape (32) consists of an adhesive layer (33) and a layer of a non-adhesive material (34) which is non-weldable to the inner side (36a) of the sheet member at the welding parameters used. In the welding process the welding tool (31) is pressed against the outer sides (1b, 2b) of the outer sheet walls and simultaneously releases heat, thereby attaching the inner side (1a) of one outer sheet wall in two respective weld lines to the outer side (36b) of the inner sheet member along the edges (36') of the sheet member, and attaching the inner side (2a) of the other outer sheet wall in two other weld lines to the outer side (36b) of the sheet member. Like in the variant of the collection bag shown in fig. 3 the two sheet walls (1, 2) may be attached to each other along their peripheries optionally in the same welding process.

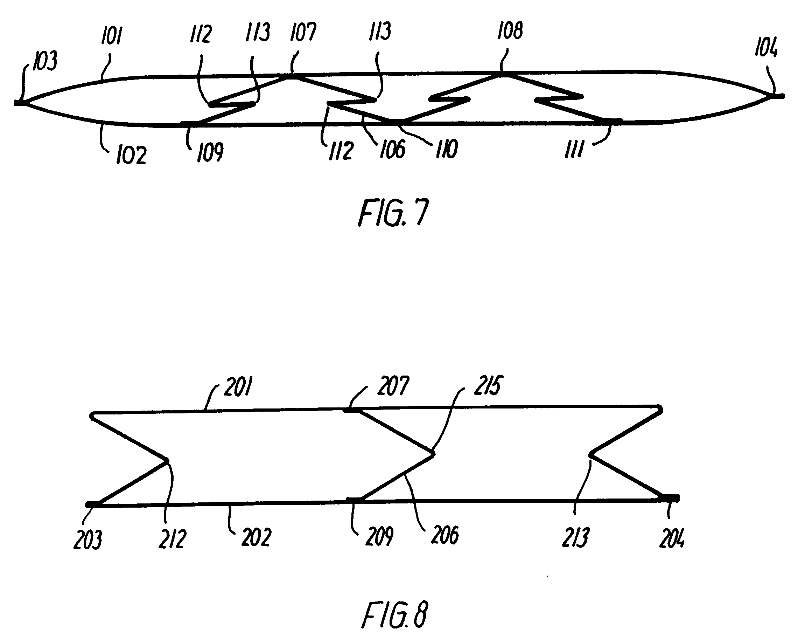

Fig. 7 shows an outline of a second embodiment of a collection bag according to the invention. The collection bag has two outer sheet walls (101, 102) which are attached to each other along their peripheries (103, 104) by e.g. glueing or welding. The two sheet walls (101, 102) have interposed between them one or more sheet members (106), and if more than one such sheet member is provided, the sheet members are attached to each other along their longitudinal edges in attachment lines (107, 108, 109, 110, 111) and thus constitute a sheet member which is attached alternately with lateral gaps to the inner sides of the one and the other, respectively, of the two outer sheet walls in the longitudinal attachment lines (107, 108, 109, 110, 111) e.g. by welding or glueing. When the bag is empty, the sheet member (106) has a free longitudinal double fold (112, 113) between each attachment line (107, 108, 109, 110, 111).

Fig. 8 shows in outline a third embodiment of a collection bag according to the invention. The collection bag has two outer sheet walls (201, 202) which are attached to each other along their peripheries (203, 204) e.g. by welding or glueing. The two sheet walls may also be provided in another manner, e.g. by a sheet tube which is joined upwardly and downwardly. At its two longitudinal edges one sheet wall has a double fold (212, 213) which folds into the bag when the bag is not filled. The two sheet walls (201, 202) have interposed between them a sheet member (206) which is attached along its side edges to the one and the other, respectively, of the two outer sheet walls (201, 202) in longitudinal attachment lines (207, 209), and which, when the bag is not filled, has a longitudinal fold (206). The bag is here shown in a half-filled, horizontal state, and it will be seen that the folds (206, 212, 213) are semi-stretched. When the bag is filled, the folds (206, 212, 213) are stretched completely and the bag maintains a plane surface.

The bags shown in figs. 7 and 8 can be produced in several different ways, as mentioned before, e.g. by glueing or welding. If the bags are welded, one of the methods described in connection with figs. 3-6 may suitably be used, and the arrangement of the various sheets, welding preventing elements and the welding tool is of course to be adapted to the bag to be produced.

## Claims

1. Collection bags for use by urine-incontinent or ostomy-operated individuals, each of which comprises a first outer sheet wall (1, 101, 201) and a second outer sheet wall (2, 102, 202), where the first outer sheet wall has an inner side (1a), said inner side (1a) facing an inner side (2a) of the second outer sheet wall when the bag is flattened, each bag having an upper end with an upper edge and a lower end with a lower edge as well as two side edges (3, 4, 103, 104, 203, 204), an inlet opening being provided in the upper end in the edge or in one of the two sheet walls (1, 2), one or more discharge openings, each of said bags having an inner sheet member or several inner sheet members (6, 16, 26, 36, 106, 206), which are joined to the inner side of the first or the second, respectively, outer sheet wall, **characterized** in that the inner sheet member or at least one of the inner sheet members (6, 16, 26, 36, 106, 206) has at least one fold (15a, 15b, 25a, 25b, 35a, 35b, 112, 113, 215) between joints (7, 8, 9, 10, 17, 18, 19, 20, 27, 28, 29, 30, 107, 108, 109, 110, 111, 206, 209) attaching the inner sheet member to the inner side (1a, 2a) of the first and the second, respectively, outer sheet walls when the bag is empty.

2. Collection bags according to claim 1, **characterized** in that the inner sheet member or at least one of the inner sheet members (6, 16, 26, 36, 106, 206) is exclusively connected with the inner sides (1a, 2a) of the outer sheet walls at respective joints spaced from the side edges (3, 4, 103, 104, 203, 204) of the outer sheet walls.

3. Collection bags according to claim 2, **characterized** in that the respective joints between the inner sheet member or the inner sheet members (6, 16, 26, 36, 106, 206) and the inner sides (1a, 2a) of the outer sheet walls terminate at a distance from the upper edge and lower edge (5) of the outer sheet walls.

4. Collection bags according to claims 1-3, **characterized** in that each bag has one or more longitudinal inward folds at its side edges (203, 204), preferably in the form of one or more pleats in one of the two outer sheet walls (201).

5. Collection bags according to claims 1-4, **characterized** in that the inner sheet member or the inner sheet members (6, 16, 26, 36, 106, 206) are connected with the inner sides (1a, 2a) of the outer sheet walls by joints to one side (13, 26b, 36b) of each individual inner sheet member.

6. Collection bags according to claims 1-5, **characterized** in that the inner sheet member or the inner sheet members (6, 16, 26, 36) together have the shape of a tube.

7. Collection bags according to claims 1-5, **characterized** in that the inner sheet member or the inner sheet members have one or more longitudinal folds or pleats (15a, 15b, 25a, 25b, 35a, 35b, 112, 113, 215).

8. Collection bags according to claims 1-7, wherein the inner sheet member or the inner sheet members (6, 16, 26, 36, 106, 206) are attached to the inner sides of the outer sheet walls and optionally to itself or each other, and wherein at least one of the attachments is made by welding, **characterized** in that the inner sheet member or at least one of the inner sheet members (6, 16, 26, 36, 106, 206) has one or more areas, preferably one side (14, 34) which is not weldable either to itself, to another inner sheet member or to the inner sides of the outer sheet walls at the used welding parameters, other inner sheet member areas being welded to the inner sides of the outer sheet walls and optionally to itself or each other.

9. Collection bags according to claim 8, **characterized** in that the non-weldable area or areas are made non-weldable by coating with a self-adhesive tape (32), and that the tape is additionally in adhesive contact with the inner sides (1a, 2a) of one of the two outer sheet walls.

10. Collection bags according to claim 8, **characterized** in that at least one of the inner sheet members (6, 16, 26, 36, 106, 206) is a laminate (16) of at least two different materials (13, 14) having mutually different welding properties.

11. Methods for producing the collection bags according to claims 1-10, wherein the inner sheet member or the inner sheet members (6, 16, 26, 36, 106, 206) are attached to the inner sides (1a, 2a) of the outer sheet walls by welding, and wherein the bag is formed by two sheet members which are welded along their peripheries (3, 4, 5, 103, 104, 203, 204) to form the outer sheet walls (1, 2, 101, 102, 201, 202), **characterized** in that all the welds are made in one and the same step or in two steps, the first step of the last-mentioned case comprising welding the inner sheet member or sheet members (6, 16, 26, 36, 106, 206) to the inner sides (1a, 2a) of the outer walls, one or more welding-preventing elements (22) being temporarily arranged, the second step comprising removing these welding-prevending elements, and then welding the outer sheet walls along their peripheries, said one or more welding-preventing elements (22) being preferably part of the welding tool (11, 21, 31).

## Patentansprüche

1. Sammelbeutel zur Verwendung durch urin-inkontinente oder harnableitungs-operierte Individuen, wobei jeder eine erste äußere Blattwand (1, 101, 201) und eine zweite äußere Blattwand (2, 102, 202) aufweist, wobei die erste äußere Blattwand eine Innenseite (1a) hat, wobei die Innenseite (1a) einer Innenseite (2a) der zweiten äußeren Blattwand zugewandt ist, wenn der Beutel flachgemacht ist, wobei jeder Beutel ein oberes Ende mit einer oberen Kante und ein unteres Ende mit einer unteren Kante sowie zwei Seitenkanten (3, 4, 103, 104, 203, 204) hat, wobei eine Einlaßöffnung in dem oberen Ende in der Kante oder in einer der beiden Blattwände (1, 2) vorgesehen ist und eine oder mehrere Auslaßöffnungen, wobei jeder der Beutel ein inneres Blattelement oder mehrere innere Blattelemente (6, 16, 26, 36, 106, 206) hat, die jeweils an der Innenseite der ersten oder der zweiten äußeren Blattwand angeschlossen sind, **dadurch gekennzeichnet**, **daß** das innere Blattelement oder zumindest eines der inneren Blattelemente (6, 16, 26, 36, 106, 206) zumindest eine Falte (15a, 15b, 25a, 25b, 35a, 35b, 112, 113, 215) zwischen Verbindungsstellen (7, 8, 9, 10, 17, 18, 19, 20, 27, 28, 29, 30, 107, 108, 109, 110, 111, 206, 209) hat, die das innere Blattelement an der Innenseite (1a, 2a) der jeweils ersten und zweiten äußeren Blattwand anbringen, wenn der Beutel leer ist.

2. Sammelbeutel nach Anspruch 1, **dadurch gekennzeichnet**, **daß** das innere Blattelement oder zumindest eines der inneren Blattelemente (6, 16, 26, 36, 106, 206) ausschließlich mit den Innenseiten (1a, 2a) der äußeren Blattwände an jeweiligen Verbindungsstellen verbunden ist, die von den Seitenkanten (3, 4, 103, 104, 203, 204) der äußeren Blattwände beabstandet sind.

3. Sammelbeutel nach Anspruch 2, **dadurch gekennzeichnet**, **daß** die jeweiligen Verbindungsstellen zwischen dem inneren Blattelement oder den inneren Blattelementen (6, 16, 26, 36, 106, 206) und den Innenseiten (1a, 2a) der äußeren Blattwände in einem Abstand von der oberen Kante und der unteren Kante (5) der äußeren Blattwand enden.

4. Sammelbeutel nach einem der Ansprüche 1-3, **dadurch gekennzeichnet**, **daß** jeder Beutel eine oder mehrere längliche Innenfalten an seinen Seitenkanten (203, 204) vorzugsweise in der Gestalt einer oder mehrerer Sicken in einer der beiden äußeren Blattwände (201) hat.

5. Sammelbeutel nach einem der Ansprüche 1-4, **dadurch gekennzeichnet**, **daß** das innere Blattelement oder die inneren Blattelemente (6, 16, 26, 36, 106, 206) mit den Innenseiten (1a, 2a) der äußeren Blattwände durch Verbindungsstellen mit einer Seite (13, 26b, 36b) von jedem einzelnen inneren Blattelement verbunden ist/sind.

6. Sammelbeutel nach einem der Ansprüche 1-5, **dadurch gekennzeichnet**, **daß** das innere Blattelement oder die inneren Blattelemente (6, 16, 26, 36) zusammen die Gestalt eines Rohrs haben.

7. Sammelbeutel nach einem der Ansprüche 1-5, **dadurch gekennzeichnet**, **daß** das innere Blattelement oder die inneren Blattelemente eine oder mehrere längliche Falten oder Sicken (15a, 15b, 25a, 25b, 35a, 35b, 112, 113, 215) haben.

8. Sammelbeutel nach einem der Ansprüche 1-7, wobei das innere Blattelement oder die inneren Blattelemente (6, 16, 26, 36, 106, 206) an der Innenseite der äußeren Blattwände und wahlweise an sich selbst oder aneinander angebracht sind, und wobei zumindest eine der Befestigungen durch Schweißen hergestellt ist, **dadurch gekennzeichnet**, **daß** das innere Blattelement oder zumindest eines der inneren Blattelemente (6, 16, 26, 36, 106, 206) einen oder mehrere Bereiche und vorzugsweise eine Seite (14, 34) hat, die mit den gebräuchlichen Schweißparametern weder mit sich selbst, noch mit einem anderen inneren Blattelement oder den Innenseiten der äußeren Blattwand verschweißbar ist, wobei andere innere Blattelementbereiche mit den inneren Seiten der äußeren Blattwände und wahlweise mit sich selbst oder miteinander verschweißt sind.

9. Sammelbeutel nach Anspruch 8, **dadurch gekennzeichnet**, **daß** der nicht schweißbare Bereich oder die nicht schweißbaren Bereiche nicht schweißbar hergestellt ist/sind, indem er/sie mit einem selbstklebenden Band (32) überzogen wird/werden, und daß das Band zusätzlich an den Innenseiten (1a, 2a) von einer der beiden äußeren Blattwände angeklebt ist.

10. Sammelbeutel nach Anspruch 8, **dadurch gekennzeichnet**, **daß** zumindest eines der inneren Blattelemente (6, 16, 26, 36, 106, 206) ein Schichtstoff (16) aus zumindest zwei verschiedenen Materialien (13, 14) mit gegenseitig unterschiedlichen Schweißeigenschaften ist.

11. Verfahren zum Herstellen der Sammelbeutel nach einem der Ansprüche 1-10, wobei das innere Blattelement oder die inneren Blattelemente (6, 16, 26, 36, 106, 206) durch Schweißen an den Innenseiten (1a, 2a) der äußeren Blattwände angebracht ist/sind, und wobei der Beutel durch zwei Blattelemente ausgebildet ist, die zur Bildung der äußeren Blattwände (1, 2, 101, 102, 201, 202) entlang ihren Rändern (3, 4, 5, 103, 104, 203, 204) verschweißt sind, **dadurch gekennzeichnet**, **daß** alle Schweißnähte in ein und demselben Schritt oder in zwei Schritten gemacht werden, wobei der erste Schritt bei dem zuletzt erwähnten Fall das Anschweißen des inneren Blattelements oder der inneren Blattelemente (6, 16, 26, 36, 106, 206) an die Innenseiten (1a, 2a) der äußeren Wand aufweist, wobei ein oder mehrere Schweißverhinderungselemente (22) vorübergehend angeordnet werden, wobei der zweite Schritt das Entfernen dieser Schweißverhinderungselemente aufweist, und wobei die äußeren Blattwände dann entlang ihren Rändern verschweißt werden, wobei das eine oder die mehreren Schweißverhinderungselemente (22) vorzugsweise ein Teil des Schweißwerkzeugs (11, 21, 31) ist/sind.

## Revendications

1. Sacs collecteurs pour utilisation par des personnes souffrant d'incontinence urinaire ou ayant subi une opération de stomie, dont chacun comprend une première (1, 101, 201) et une deuxième (2, 102, 202) parois à feuille externes, la première paroi à feuille externe ayant un côté interne (1a), ce côté interne (1a) faisant face à un côté interne (2a) de la deuxième paroi externe lorsque le sac est aplati, chaque sac ayant une extrémité supérieure avec un bord supérieur et une extrémité inférieure avec un bord inférieur, ainsi que deux bords latéraux (3, 4, 103, 104, 203, 204), un orifice d'entrée étant prévu dans l'extrémité supérieure, dans le bord ou dans l'une des deux parois (1, 2), et un ou plusieurs orifices de décharge, chacun desdits sacs ayant un ou plusieurs élément(s) à feuille interne(s) (6, 16, 26, 36, 106, 206), qui sont réunis au côté interne respectivement de la première ou de la seconde paroi à feuille extérieure, caractérisés en ce que l'élément à feuille interne ou au moins l'un des éléments à feuille internes (6, 16, 26, 36, 106, 206) a au moins une pliure (15a, 15b, 25a, 25b, 35a, 35b, 112, 113, 215) entre des points de liaison (7, 8, 9, 10, 17, 18, 19, 20, 27, 28, 29, 30, 107, 108, 109, 110, 111, 206, 209) reliant l'élément à feuille interne aux côtés internes (1a, 2a) respectivement, des première et deuxième parois à feuille externes lorsque le sac est vide.

2. Sacs collecteurs selon la revendication 1, caractérisés en ce que l'élément à feuille interne ou au moins l'un des éléments à feuille internes (6, 16, 26, 36, 106, 206) est exclusivement réuni aux côtés internes (1a, 2a) des parois à feuille externes en des points de liaison respectifs, espacés des bords latéraux (3, 4, 103, 104, 203, 204) des parois à feuille externes.

3. Sacs collecteurs selon la revendication 2, caractérisés en ce que les points de liaison respectifs entre l'élément ou les éléments à feuille internes (6, 16, 26, 36, 106, 206) et les côtés internes (1a, 2a) des parois à feuille externes se terminent à une certaine distance du bord supérieur et du bord inférieur (5) des parois à feuille externes.

4. Sacs collecteurs selon les revendications 1-3, caractérisés en ce que chaque sac a une ou plusieurs pliures longitudinales vers l'intérieur au niveau de ses bords latéraux (203, 204), de préférence sous la forme d'un ou de plusieurs plis dans l'une des deux parois à feuille externes (201).

5. Sacs collecteurs selon les revendications 1-4, caractérisés en ce que l'élément ou les éléments à feuille internes (6, 16, 26, 36, 106, 206) sont reliés aux côtés internes (1a, 2a) des parois à feuille externes par des points de liaison d'un côté (13, 26b, 36b) de chaque élément à feuille interne individuel.

6. Sacs collecteurs selon les revendications 1-5, caractérisés en ce que l'élément ou les éléments à feuille internes (6, 16, 26, 36) ont ensemble la forme d'un tube.

7. Sacs collecteurs selon les revendications 1-5, caractérisés en ce que l'élément interne ou les éléments à feuille internes ont une ou plusieurs pliures longitudinales ou plis (15a, 15b, 25a, 25b, 35a, 35b, 112, 113, 215).

8. Sacs collecteurs selon les revendications 1-7, dans lesquels l'élément ou les éléments à feuille internes (6, 16, 26, 36, 106, 206) sont réunis aux côtés internes des parois à feuille externes et éventuellement à elle-même, ou les uns aux autres, et dans lesquels au moins l'un des points de liaison est effectué par soudage, caractérisés en ce que l'élément à feuille interne ou au moins l'un des éléments à feuille internes (6, 16, 26, 36, 106, 206) a une ou plusieurs zones, de préférence un côté (14, 34) qui n'est soudable ni à luimême, ni à un autre élément à feuille interne ou aux côtés internes des parois à feuille externes avec les paramètres de soudage utilisés, d'autres zones de ces éléments à feuille internes étant soudées aux côtés internes des parois à feuille externes et éventuellement à elle-même, ou les unes aux autres.

9. Sacs collecteurs selon la revendication 8, caractérisés en ce que la zone ou les zones non soudables sont rendues non soudables par un revêtement de ruban auto-adhésif (32), et en ce que ce ruban est de plus en contact adhésif avec les côtés internes (1a, 2a) de l'une des deux parois à feuille externes.

10. Sacs collecteurs selon la revendication 8, caractérisés en ce qu'au moins un des éléments à feuille internes (6, 16, 26, 36, 106, 206) est constitué d'un stratifié (16) formé d'au moins deux matériaux différents (13, 14), ayant des propriétés de soudage mutuellement différentes.

11. Méthode de production des sacs collecteurs selon les revendications 1-10, dans laquelle l'élément ou les éléments à feuille internes (6, 16, 26, 36, 106, 206) sont reliés aux côtés internes (1a, 2a) des parois à feuille externes par soudage, et dans laquelle chaque sac est formé par deux éléments à feuille soudés le long de leur périphérie (3, 4, 5, 103, 104, 203, 204) pour constituer les parois à feuille externes (1, 2, 101, 102, 201, 202), caractérisée en ce que toutes les soudures sont réalisées en une seule et même étape ou en deux étapes, la première étape du cas mentionné en dernier comprenant le soudage de l'élément ou des éléments à feuille internes (6, 16, 26, 36, 106, 206) aux côtés internes (1a, 2a) des parois externes, un ou plusieurs organes empêchant le soudage (22) étant prévus temporairement, la deuxième étape comprenant le retrait de ces organes, puis le soudage des parois à feuille externes le long de leur périphérie, un ou plusieurs de ces organes (22) faisant de préférence partie de l'outil de soudage (11 ,21, 31).
